Europäisches Patentamt

European Patent Office

Office européen des brevets

(11) Veröffentlichungsnummer: **0 063 640**
**A2**

(12) **EUROPÄISCHE PATENTANMELDUNG**

(21) Anmeldenummer: **81109364.0**

(22) Anmeldetag: **30.10.81**

(51) Int. Cl.³: **A 61 M 5/00**

(30) Priorität: **24.04.81 DE 8112180 U**

(71) Anmelder: **INTERMEDICAT GMBH, Gerliswilstrasse 43, CH-6020 Emmenbrücke (CH)**

(43) Veröffentlichungstag der Anmeldung: **03.11.82**
**Patentblatt 82/44**

(72) Erfinder: **Scherer, Jürgen, Birnheck 12, D-6233 Kelkheim 6 (DE)**

(84) Benannte Vertragsstaaten: **AT BE CH DE FR GB IT LI LU NL SE**

(74) Vertreter: **von Kreisler, Alek et al, Patentanwälte Von Kreisler-Schönwald-Fues-Keller Selting-Werner Deichmannhaus am Hauptbahnhof, D-5000 Köln 1 (DE)**

(54) Verschlusskörper für die Leitungen medizinischer Geräte.

(57) Ein Verschlußkörper für die Leitungen medizinischer Geräte weist einen konischen Stopfen (16) auf, der von einem zylindrischen Mantel (18) mit radialem Abstand umgeben ist. An der dem Stopfen (16) abgewandten Seite befindet sich ein Hohlstutzen (20). Der Verschlußkörper eignet sich für den Anschluß an männliche oder weibliche Verbindungsstücke, an denen entweder der Hohlstutzen (20) oder der Stopfen (16) als Verschlußelement benutzt wird.

Verschlußkörper für die Leitungen medizinischer Geräte

Die Erfindung betrifft einen Verschlußkörper für die Leitungen medizinischer Geräte, mit in unterschiedliche Richtungen weisenden Verschlußteilen.

Im medizinischen Bereich werden eine große Anzahl Geräte eingesetzt, die z.B. der Flüssigkeitsleitung oder der Entnahme von Körperflüssigkeiten oder von Gewebeproben dienen. Ein Teil dieser Geräte wird aus hygienischen Gründen meist aus Kunststoffen zum einmaligen Gebrauch hergestellt und sterilisiert in den Handel gebracht. Es ist üblich, Verbindungen an diesen Einmalgeräten mit Hilfe zusammenpassender konischer Verbindungsstücke herzustellen, wobei jeweils ein Innenkonus und ein Außenkonus zusammengesteckt oder zusammengeschraubt werden. Durch mäßigen Kraftaufwand lassen sich die Verbindungen wieder trennen und erlauben somit die unterschiedlichsten Kombinationen medizinischer Geräte, die während einer Behandlung notwendig werden. Die Verbindungsstücke haben zentrisch in Längsrichtung verlaufend eine Bohrung, die den ankommenden und weiterführenden Leitungsweg durchgängig macht. Beispiele für
medizinische Geräte mit Kegelverbindungen sind

- Injektionsspritzen und Kanülen zur Injektion von
  Medikamenten oder zur Blutentnahme,
- Katheter zur Einleitung von Infusionslösungen und
  Blutkonserven in das Blutgefäßsystem,
- Sonden für diagnostische Zwecke in der Kardiologie
  und in der Urologie,
- Übertragungsgeräte für Infusionslösungen zur parenteralen Therapie,
- Endoskope zur Untersuchung innerer Organe,
- gummigedichtete Zugabestellen in Schlauchsystemen,
- Ein- und Mehrwegehähne.

Während der therapeutischen oder diagnostischen Behandlung
eines Patienten kommt es nicht selten vor, daß durch Verbindungsstücke gekoppelte Gas- oder Flüssigkeitsleitungen getrennt werden müssen, um weitere Maßnahmen vorzunehmen, ohne daß dabei der im Patienten bzw.
in dessen Blutgefäßen liegende Leitungsteil, z.B. ein
Katheter, entfernt werden kann oder für die weitere Behandlung sogar an seiner Stelle verbleiben muß. Das vom
Patienten wegweisende (distale) Katheterende, der sogenannte Katheteransatz, darf während der Leitungsunterbrechung nicht offen bleiben. Verschlossen werden auch die an
Spritzen vorhandenen Verbindungsstücke, wenn der Inhalt
der Spritze vor einer Injektion oder nach der Füllung transportiert oder für eine Zeit gelagert werden muß und nicht
dem Gasaustausch mit der Atmosphäre ausgesetzt sein soll.
Letzteres ist bei der Blutgasanalyse üblich, wobei es
weiterhin von Wichtigkeit ist, daß der Verschlußkörper praktisch keinen Luftraum enthält, aus dem ein die Situation beeinflussendes Analyseresultat sich ergeben könnte.

Es sind Verschlußkörper bekannt, die als unterschiedlich geformte Kappen mit Hohlstutzen ausgebildet und daher zum Verschließen von Außenkegeln geeignet sind. Das Gegenstück hierzu sind massiv oder hohl geformte Verschlußkörper mit Stopfen, die ihrerseits in der Lage sind, in Innenkegel eingesetzt zu werden und diese zu verschließen. Da im medizinischen Bereich konische Stopfen mit unterschiedlichen Abschrägungen von 6% oder 10% üblich sind (sog. Luer- oder Rekordkegel), gibt es auch linear angeordnete einteilige Kombinationen aus diesen beiden Stopfen, von denen jeweils der eine oder andere als Verschluß eingesetzt wird.

Aus Sicherheitsgründen werden in medizinisch verwendeten Leitungssystemen und Geräten zunehmend anstelle der lange Zeit üblich gewesenen einfachen Kegelsteckverbindungen verriegelbare Kegelverbindungen benutzt. Für solche verriegelbaren Innen- und Außenkegel gibt es jedoch keine geeigneten Verschlußkörper,mit denen die betreffenden Leitungen entsprechend den medizinischen Notwendigkeiten temporär verschlossen werden können.

Der Erfindung liegt die Aufgabe zugrunde, einen Verschlußkörper der eingangs genannten Art zu schaffen, der sowohl zum Verschließen von lediglich steckbaren Kegelansätzen als auch zum Verschließen von verriegelbaren Kegelansätzen geeignet ist - gleichgültig, ob diese Kegelansätze als Innenkegel oder Außenkegel ausgebildet sind.

Zur Lösung dieser Aufgabe ist erfindungsgemäß vorgesehen, daß eines der Verschlußteile ein Stopfen ist, der auf mindestens einem Teil seiner Länge mit Abstand von einem zylindrischen Mantel umgeben ist, dessen Innenwand erste Verriegelungselemente aufweist, und daß das andere Verschlußteil ein Hohlstutzen ist, von dessen Außenwand zweite Verriegelungselemente abstehen.

Ein derartiger Verschlußkörper kann für verschiedenartige Verbindungsstücke benutzt werden, nämlich

a) für Verbindungsstücke mit verriegelbarem Innenkegel,
b) für Verbindungsstücke mit verriegelbarem Außenkegel,
c) für Verbindungsstücke mit lediglich steckbarem Innenkegel und
d) für Verbindungsstücke mit lediglich steckbarem Außenkegel.

Voraussetzung ist selbstverständlich, daß die Form und Größe des Stopfens bzw. des Hohlstutzens auf die entsprechenden Abmessungen des an der Leitung befestigten Verbindungsstückes abgestimmt sind.

Obwohl der Stopfen zweckmäßigerweise aus einem Vollmaterial besteht, kann er auch im wesentlichen hohl ausgebildet sein, wobei sein vorderes freies Ende durch eine Trennwand verschlossen ist.

In vorteilhafter Ausgestaltung der Erfindung sind die beiden Verschlußteile des Verschlußkörpers einander derart angepaßt, daß zwei gleiche Verschlußkörper durch Ineinanderstecken ihrer Verschlußteile miteinander verriegelbar sind. Zwar werden zwei Verschlußkörper nicht unmittelbar aneinandergesetzt, jedoch wird durch die zueinander passende Bemessung ihrer Verschlußteile erreicht, daß beide Verschlußteile für ein ganz bestimmtes Verbindungssystem mit festgelegten Abmessungen der Verbindungsstücke anwendbar sind, und zwar entweder für Außenkegel oder für Innenkegel dieses Verbindungssystems.

Vorzugsweise entspricht die Tiefe der Ausnehmung des Hohlstutzens annähernd der Länge des Stopfens. Da der Stopfen in seinen Abmessungen dem Außenkegel der zugehörigen Verbindungsstücke und der Hohlstutzen dem Innenkegel der zugehörigen Verbindungsstücke entspricht, ist ein derartiger Verschlußkörper so gestaltet, daß bei seiner Anwendung der Luftraum innerhalb der zusammenwirkenden Teile des an der Leitung befestigten Verbindungsstückes und des Verschlußkörpers so klein wie möglich gehalten wird.

Um den Verschlußkörper griffig zu machen, was bei der Verwendung im medizinischen Bereich bei Anwesenheit von Körperflüssigkeiten oft erforderlich ist, können auf der Außenseite des Verschlußkörpers entsprechende Profilierungen wie die Rändel, Griffmulden und polygonale Flächen geformt sein.

In der Verwendung von Werkstoffen für derartige Teile bestehen keine Beschränkungen, sie können üblicherweise aus Metall und Kunststoffen wie Polyethylen, Polypropylen, Polystyrolhomo- und -copolymerisate, Polyamide, Polyvinylchlorid, Polycarbonat sein, wobei den Kunststoffen zur Herstellung von Teilen zum einmaligen Gebrauch der Vorzug gegeben wird.

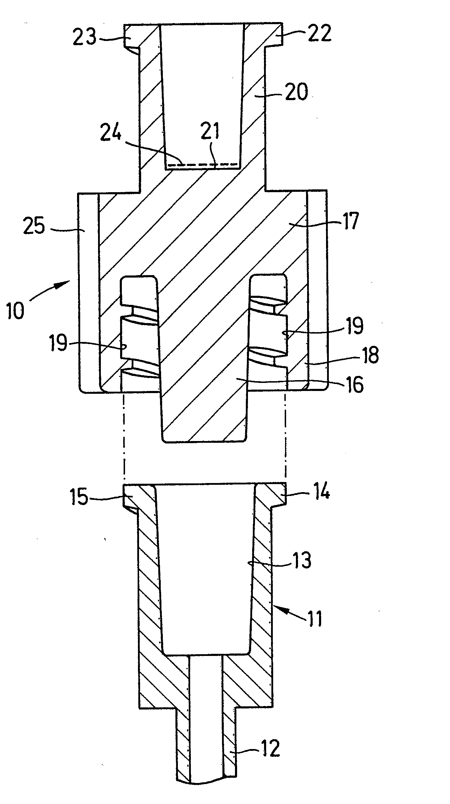

Im folgenden wird unter Bezugnahme auf die einzige Figur der Zeichnung ein Ausführungsbeispiel der Erfindung näher erläutert.

In der Zeichnung ist ein Verschlußkörper im Längsschnitt dargestellt.

Der Verschlußkörper 10 dient zum abdichtenden Verschliessen des Verbindungsstückes 11 einer Schlauchleitung 12. Bei dem dargestellten Ausführungsbeispiel weist das Verbindungsstück 11 einen Innenkegel 13 auf, von dessen äußeren Rand Verriegelungselemente 14,15 radial abstehen. Die Verriegelungselemente 14,15 sind in bekannter Weise leicht schraubenförmig schräggestellt und sie erstrecken sich jeweils nur über ein kurzes Stück des Umfangs des Verbindungsstückes 11.

Der Verschlußkörper 10 weist einen Stopfen 16 auf, der in Form und Länge dem Innenkegel 13 im wesentlichen angepaßt ist. Der Stopfen 16 ist an seiner Rückseite mit einem Basisteil 17 verbunden, von dem koaxial zu dem Stopfen 16 ein Mantel 18 absteht, der den Stopfen 16 auf einem Teil der Länge dieses Stopfens mit radialem Abstand umgibt. An der Innenseite des Mantels 18 befinden sich die ersten Verriegelungselemente 19 in Form schraubenförmiger Nuten. Beim Ansetzen des Verschlußkörpers 10 an das Verbindungsstück 11 greifen die radial abstehenden Verriegelungselemente 14,15 des Verbindungsstückes 11 in die Nuten 19 des Verschlußkörpers 10 ein. Durch Drehen des Verschlußkörpers 10 wird der massive Stopfen 16 in den Innenkegel 13 eingeschoben, wobei der axiale Vorschub während des Drehens durch die Steigung der Nuten 19 bestimmt wird. Von der dem Stopfen 16 abgewandten Seite des Basisteiles 17 steht der Hohlstutzen 20 ab. Der Hohlstutzen 20 ist in gleicher Weise ausgebildet wie das Verbindungsstück 11, hat jedoch keinen axialen Durchlaß. Die Stirnseite 21 des Hohlstutzens 20 ist vielmehr verschlossen. Der Hohlstutzen 20 hat eine zylindrische Außenfläche und eine kegelstumpfförmige Innenfläche. An dem äußeren Ende des Hohlstutzens 20 befinden sich die radial abstehenden zweiten Verriegelungselemente 22,23, die in gleicher Weise ausgebildet sind wie die Verriegelungselemente 14 und 15 des Verbindungsstückes 11.

Der Hohlstutzen 20 bildet ein Verschlußteil, das zum Verschließen eines (nicht dargestellten) Verbindungsstückes benutzt werden kann, das in gleicher Weise ausgebildet ist wie die untere Hälfte des Verschlußkörpers 10, jedoch einen in Längsrichtung durchgehenden axialen Kanal aufweist. Auf diese Weise kann der Verschlußkörper 10 sowohl zum Verschließen eines Verbindungsstückes 11 mit Innenkonus 13 als auch zum Verschließen eines im wesentlichen komplementär geformten (nicht dargestellten) Verbindungsstückes mit Außenkegel benutzt werden.

Die Tiefe des Hohlstutzens 20 ist so bemessen, daß ein in ihn eingesetzter Außenkegel bis nahe an die Stirnwand 21, z.B. bis zu der gestrichelten Linie 24, vordringt. Damit ergibt sich ein nur sehr kleines Restvolumen innerhalb des Verschlußkörpers.

Das Basisteil 17 und der Mantel 18 sind an ihrer Außenseite mit einer Profilierung 25 versehen, die das Angreifen am Verschlußkörper mit der Hand erleichtert.

Der gesamte Verschlußkörper ist einstückig, z.B. als Spritzgußteil, hergestellt. Er kann für Verbindungsstücke benutzt werden, die Verriegelungselemente 14,15 aufweisen, aber auch für solche Verbindungsstücke, die keine Verriegelungselemente haben und bei denen die Klemmung ausschließlich durch Eindrücken des konischen Verschlußstopfens 16 in den Innenkegel oder durch Eindrücken eines Außenkegels in die konische Innenwand des Hohlstutzens
20 erfolgt.

A N S P R Ü C H E

1. Verschlußkörper für die Leitungen medizinischer Geräte, mit in unterschiedliche Richtungen weisenden Verschlußteilen, d a d u r c h  g e k e n n - z e i c h n e t ,  daß eines der Verschlußteile ein Stopfen (16) ist, der auf mindestens einem Teil seiner Länge mit Abstand von einem zylindrischen Mantel (18) umgeben ist, dessen Innenwand erste Verriegelungselemente (19) aufweist, und daß das andere Verschlußteil ein Hohlstutzten (20) ist, von dessen Außenwand zweite Verriegelungselemente (22,23) abstehen.

2. Verschlußkörper nach Anspruch 1, dadurch gekennzeichnet, daß die beiden Verschlußteile derart einander angepaßt sind, daß zwei gleiche Verschlußkörper (10) durch Ineinanderstecken ihrer Verschlußteile miteinander verriegelbar sind.

3. Verschlußkörper nach Anspruch 1 oder 2, dadurch gekennzeichnet, daß die Tiefe der Ausnehmung des Hohlstutzens (20) annähernd der Länge des Stopfens (16) entspricht.